# EUROPÄISCHE PATENTANMELDUNG

(11) **EP 1 995 679 A1**
(43) Veröffentlichungstag der Anmeldung: **26.11.2008**
(21) Anmeldenummer: 07010474.0
(22) Anmeldetag: 25.05.2007
(51) Int. Cl.: G06F 19/00

(54) **Vorrichtung und Verfahren zur zentralen Überwachung und/oder Steuerung wenigstens eines Gerätes**

(71) Anmelder: Karl Storz GmbH & Co. KG, 78532 Tuttlingen (DE)
(72) Erfinder: Skupin, Horst, 78048 VS-Villingen (DE)
(74) Vertreter: Fugmann, Winfried

(57) **Zusammenfassung**

Um eine allgemein verfügbare Datenbasis von vorbestimmten Gerätekonfigurationen und -parametern bei medizinischen Eingriffen nutzen zu können, wird eine Vorrichtung zur Steuerung und/oder Überwachung mindestens eines Gerätes bei einem medizinischen Eingriff vorgeschlagen, die mindestens eine Steuerungseinheit zur Steuerung und/oder Überwachung des mindestens einen Gerätes und mindestens eine Anzeigeeinheit zur Anzeige von Daten umfasst, wobei ein Zugriff möglich ist auf eingriffsspezifische Daten, die zumindest Daten über die bei einem vorgegebenen Eingriff bei dem mindestens einen Gerät einzustellenden Parameterwerte umfassen, wobei ferner die Parameterwerte zumindest teilweise benutzerunabhängig vorbestimmt sind, die Parameterwerte von einem Benutzer zur Anzeige mittels der Anzeigeeinheit aufgerufen werden können, und wobei ein Bestätigungselement vorgesehen ist, durch dessen Betätigung die vorbestimmten Parameterwerte für das mindestens eine Gerät übernommen werden.

## Beschreibung

Die vorliegende Erfindung betrifft eine Vorrichtung und ein Verfahren zur zentralen Überwachung und/oder Steuerung wenigstens eines Geräts, das während eines medizinischen Eingriffs benutzt wird, mit mindestens einer Steuerungseinheit zur Steuerung und/oder Überwachung des mindestens einen Gerätes und mindestens einer Anzeigeeinheit zur Anzeige von Daten, wobei ein Zugriff möglich ist auf eingriffsspezifische Daten, die zumindest Daten über die bei einem vorgegebenen Eingriff bei dem mindestens einen Gerät einzustellenden Parameterwerte umfassen.

Mit der Zunahme der Anzahl und der Komplexität der bei medizinischen Eingriffen jeder Art, insbesondere bei minimal-invasiven chirurgischen Operationen, aber auch diagnostischen oder nichtinvasiven therapeutischen Verfahren, zum Einsatz kommenden Geräte sind auch die Anforderungen an die Bedienung durch den verantwortlichen Arzt gewachsen. Zur Erleichterung der Bedienung, zur Erhöhung der Sicherheit und zur Verbesserung von Dokumentation und Verwaltungsabläufen sind deshalb Systeme geschaffen worden, bei denen über eine oder mehrere Steuerungseinheiten eine Vielzahl von medizinischen und nichtmedizinischen Geräten, die bei einer Operation zum Einsatz kommen, bedient werden können.

Derartige Einrichtungen zur zentralen Überwachung und/oder Steuerung von bei einer Operation zum Einsatz kommenden Geräten sind beispielsweise aus den Druckschriften DE 92 18 373 U1, EP 1 034 480 B1 oder WO 02/19957 bekannt, auf die zur Erläuterung aller hier nicht näher beschriebenen Einzelheiten ausdrücklich Bezug genommen wird. Aus der DE 199 04 090 A1 ist bekannt, die einzelnen Geräte und einen Leitrechner über einen CAN-Bus miteinander zu verbinden, wobei die einzelnen Geräte als Slaves und der Leitrechner als Master dienen; über den Leitrechner sind alle Geräte steuerbar.

Da die Funktion der verwendeten Geräte durch verschiedene Parameter bestimmt wird, wie beispielsweise Insufflationsdruck, HF-Spannung usw., ist es vorgesehen, dass diese Parameter über eine Eingabeeinheit, beispielsweise einen Touch Screen, eingegeben werden können; ebenso ist es auch bekannt, dass die vom Arzt eingegebenen Geräteparameter in einem Speicher gespeichert und aus diesem zur Verwendung bei einer späteren Operation abgerufen werden können. Ein solches System zur Steuerung von Einrichtungen, die bei einer chirurgischen Operation verwendet werden, wird beispielsweise von der Fa. KARL STORZ unter der Bezeichnung "OR1®" angeboten (s. www.karlstorz.de).

Aus den Druckschriften US 6 708 184 und US 6,928,490 ist es bekannt, für den Arzt während der Operation Informationen aus dem Internet verfügbar zu machen, beispielsweise einen digitalen anatomischen Atlas. Ebenso ist aus der US 5,819,229 die Verwendung von Datenbanken im Rahmen eines integrierten Operationsassistenz- und Überwachungssystems bekannt, die zur Archivierung der Patienteninformationen und der Registrierung der während der Operation anfallenden Parameter eingesetzt werden. Die in den Datenbanken abgespeicherten Informationen können dabei jedoch nicht zur direkten Steuerung der medizinischen Geräte eingesetzt werden, die während des chirurgischen Eingriffs verwendet werden.

Aus der DE 697 34 592 T2 ist ein System zur Steuerung ophthalmologischer chirurgischer Eingriffe bekannt, das mit mikrochirurgischen Instrumenten verbundene chirurgische Module, einen Datenübertragungsbus und eine Benutzerschnittstelle aufweist. Die Benutzerschnittstelle enthält eine Verarbeitungseinrichtung und einen Speicher zur Speicherung einer Vielzahl an Geräteparametern für die mikrochirurgischen Instrumente. Durch die Verarbeitungseinrichtung kann eine Gruppe von Parametern abgerufen und über den Datenübertragungsbus auf die chirurgischen Module übertragen werden, welche die mikrochirurgischen Instrumente in Abhängigkeit von der aus dem Speicher abgerufenen Gruppe der Parameter steuern. Die Benutzerschnittstelle weist ein Menü auf, in dem der jeweilige behandelnde Chirurg, beispielsweise unter seinem Namen in einem Chirurgenauswahlmenü, die Geräteparameter der mikrochirurgischen Instrumente zu gesonderten Gruppen zusammenstellen kann, die er zur Durchführung eines spezifischen operativen Eingriffs als vorteilhaft erachtet. Diese gesonderten Gruppen an Parametern sind in Untermenüs zu dem übergeordneten Chirurgen-Auswahlmenü angelegt und werden zur Durchführung eines bestimmten Eingriffs vom Chirurgen entsprechend ausgewählt. Auch aus den Druckschriften US 5,788,688 und US 6,397,286 ist es bekannt, dass der Chirurg von ihm zuvor für bestimmte Eingriffe definierte und in einem medizinischen Steuerungssystem gespeicherte, bevorzugte Gerätekonfigurationen situationsabhängig abrufen und verwenden kann.

Der Nachteil hierbei ist, dass der Chirurg für eine bestimmte chirurgische Prozedur nicht auf im System verfügbare Parameterwerte und Gerätekonfigurationen zurückgreifen kann, die sich bei einer Vielzahl von chirurgischen Eingriffen der gleichen Art bewährt haben. Stattdessen muss er selbst die bei einem bestimmten medizinischen Eingriff notwendigen Geräte zusammenstellen und die optimalen Parametereinstellungen hierfür im System implementieren, um darauf im konkreten Fall zurückgreifen zu können. Dies ist jedoch zeitaufwendig und potentiell fehlerträchtig, da nicht jeder Arzt und nicht jede Klinik über die Erfahrung verfügen kann, die notwendig ist, um für alle Arten von Eingriffen und für alle in der Praxis auftretenden Situationen die optimalen Konfigurationen und Parameterwerte zu ermitteln.

Es wäre deshalb wünschenswert, eine allgemein verfügbare Datenbasis von vorbestimmten, vom jeweiligen Arzt unabhängigen, in einer Vielzahl von Operationen als optimal erwiesenen und von anerkannten Fachleuten ermittelten Gerätekonfigurationen und -parametern bei medizinischen Eingriffen nutzen zu können. Andererseits können auch solche vorbestimmten Parameterwerte nicht für jeden Einzelfall und nicht für jeden Patienten die optimalen sein. Deshalb ist es aus Sicherheitsgründen unabdingbar, dass der jeweilige behandelnde Arzt vor einem Eingriff positive Kenntnis von den einzustellenden Geräteparametern nimmt und diese im Hinblick auf die konkrete Situation akzeptiert. Es ist jedoch nicht ohne weiteres auszuschließen, dass dies bei immer wiederkehrenden Routineeingriffen oder auch in den bei Operationen immer wieder vorkommenden Notfall- und Stresssituationen versäumt werden könnte.

Es ist daher Aufgabe der vorliegenden Erfindung, ein System und ein Verfahren zur Steuerung und/oder Überwachung von Geräten bei medizinischen Eingriffen anzugeben, bei dem einerseits ein Zugriff auf allgemein gültige, in einer Vielzahl von vergleichbaren Eingriffen als optimal herausgestellte Gerätekonfigurationen und -parameter möglich ist, andererseits aber auch sichergestellt ist, dass der Arzt bei jedem einzelnen Eingriff positive Kenntnis von diesen genommen hat.

Diese Aufgabe wird bei einer Vorrichtung der eingangs genannten Art durch die Merkmale des Anspruchs 1 und bei einem erfindungsgemäßen Verfahren durch die Merkmale des Anspruchs 16 gelöst.

Dadurch, dass ein Zugriff auf vorbestimmte, nicht von dem jeweiligen Benutzer eingegebene, Parameterwerte für die bei einer bestimmten Art von Operation verwendeten Geräte vorgesehen ist, und dass mindestens ein Bestätigungselement vorgesehen ist, dessen Betätigung zur Übernahme der vorgegebenen Parameterwerte durch die Geräte notwendig ist, ist sichergestellt, dass der verantwortliche Arzt einerseits auf vorgegebene allgemeingültige, bei einer Vielzahl von vergleichbaren Operationen bewährte Parameter zurückgreifen kann, aber auch gezwungen ist, hiervon Kenntnis zu nehmen. Durch die Anzeigeeinheit werden dem behandelnden Arzt die Parameter, die von ihm aufgerufen wurden, zur kritischen Bewertung und Freigabe dargestellt. Das Bestätigungselement erfordert eine aktive Betätigung durch den Arzt als verantwortlichen Nutzer des Systems und zwingt ihn damit zu einer bewussten Bestätigung der zu übernehmenden Parameterwerte.

Das Bestätigungselement überlässt dem behandelnden Arzt die Wahl, den ausgewählten Parametereinstellungen zuzustimmen, was die Freigabe der Parameter bzw. die Übernahme der Parameterwerte durch die Geräte bewirkt, oder diese abzulehnen. Dies wird beispielsweise dadurch erreicht, dass durch die Ausgabevorrichtung des Systems an den behandelnden Arzt die Frage gerichtet wird: ,,Stimmen Sie den ausgewählten Parameterwerten zu?". Der behandelnde Arzt kann durch entsprechende Betätigung des Bestätigungselements die Frage dann bejahen oder verneinen. Der behandelnde Arzt ist daher gezwungen, sich Rechenschaft über die Eignung der aus der Datenbank übernommenen Parametereinstellungen zur Durchführung des spezifischen Eingriffs am betreffenden Patienten abzulegen.

Darüber hinaus hat der Arzt gemäß einer bevorzugten Ausführungsform der Erfindung die Möglichkeit, die Werte einzelner Parameter, die durch die Anzeigeeinheit dargestellt werden, zu verändern und dann der Gesamtheit der, teilweise veränderten und teilweise unveränderten Parametereinstellungen durch Betätigen des Bestätigungselements zuzustimmen, was die Freigabe der Parameter bzw. die Übernahme der Werte der eingestellten Parameter durch die verwendeten Geräte auslöst. Dies hat den Vorteil, dass der Arzt nicht gezwungen ist, die vorbestimmten Parameterwerte unverändert zu übemehmen, sondern diese gemäß seinem Ermessen an die jeweiligen Situation und/oder den jeweiligen Patienten anpassen kann. So erfordern beispielsweise Operationen an Kindern, adipösen Patienten oder Patienten, die besonderen Risikogruppen angehören, eine spezifische Anpassung der Gerätekonfigurationen und Parameterwerte.
Da auch die Art und Anzahl der bei einem bestimmten Eingriff notwendigen Geräte allgemeingültigen Überlegungen unterliegt, ist es gemäß einer weiteren bevorzugten Ausführungsform vorgesehen, dass die vorbestimmten, eingriffsspezifischen Daten auch vorbestimmte Daten über die bei dem vorgegebenen Eingriff zum Einsatz kommende Gerätekonfiguration umfassen können. Hiermit ist sichergestellt, dass dem behandelnden Arzt in jedem Fall die für den betreffenden Eingriff optimale Zusammenstellung von Geräten vorgeschlagen wird, für die, oder zumindest für einige dieser Geräte, dann auch die vorbestimmten Parameterwerte angezeigt werden und er diese freigeben oder ändern kann.

Gemäß einer weiteren bevorzugten Ausführungsform der Erfindung werden die vorbestimmten, ggf. vom Benutzer geänderten Parameterwerte nach Betätigung des Bestätigungselements an das bzw. die bei dem Eingriff verwendeten Geräte übermittelt, die dann die betreffende Einstellung übernehmen. Dies kann beispielsweise durch ein an sich bekanntes Bus-System geschehen und stellt eine einfache und sichere Lösung dar.

Alternativ hierzu könnten die vorbestimmten und ggf. geänderte Parameterwerte auch schon vor Betätigung des Bestätigungselements, beispielsweise nach dem Aufruf der vorbestimmten Parameter durch den Benutzer oder gleichzeitig mit der Anzeige der vorbestimmten Parameter auf der Anzeigeeinheit bzw. mit einer Änderung durch den Benutzer an die bei dem Eingriff zur Verwendung kommenden Geräte übermittelt werden. Nach Betätigung des Bestätigungselements ist dann lediglich noch ein Aktivierungssignal zu übermitteln, das die Übernahme der bereits übertragenen Daten veranlasst. Diese Vorgehensweise ist vorteilhaft, wenn beispielsweise größere Datenmengen zu übertragen sind oder die Übernahme der Parameter durch die angeschlossenen Geräte einen größeren Zeitbedarf hat.

In beiden Fällen können die angeschlossenen Geräte die durch die übertragenen Parameter bestimmte Aktion, wie beispielsweise die Erzeugung einer bestimmten Lichtleistung, HF-Spannung oder eines bestimmten Drucks, sofort ausführen oder lediglich in Bereitschaft hierzu versetzt werden, wobei die Aktion erst durch ein weiteres, ggf. durch einen Sensor automatisch erzeugtes Signal ausgelöst wird.

Gemäß einer weiteren bevorzugten Ausführungsform löst die Betätigung des Bestätigungselements eine Speicherung der ausgewählten und freigegebenen Gerätekonfiguration und Geräteparameter aus. Hierdurch werden die Anforderungen an eine umfassende Dokumentation im Zusammenhang mit chirurgischen Eingriffen erfüllt. Hierbei kann auch eine elektronische Patientendatei geöffnet werden, der nachfolgend entstehende Daten über den Betrieb der verwendeten Geräte, aber auch beispielsweise Bilddaten aus der Operation, zugeordnet werden. Gleichzeitig können weitere Aktionen, wie beispielsweise der Eintrag eines entsprechenden Textbausteins in ein OP-Berichtsformular oder die Nachbestellung von Verbrauchsmaterialien, ausgelöst werden.

In einer weiteren bevorzugten Ausführungsform ist das Bestätigungselement als Schalter, beispielsweise als Knopf, Kippschalter, Schiebeschalter oder Schloss mit Freigabeschlüssel oder o.ä. ausgebildet, wobei ein Kontrollelement vorgesehen ist, das bei Betätigung des Bestätigungselements ein wahrnehmbares Signal abgibt, beispielsweise eine Kontrollleuchte, die aufleuchtet, oder ein akustisches Element, das einen Kontrollton erzeugt. Diese Maßnahme hat den Vorteil, dass sie besonders kostengünstig und einfach realisiert werden kann und dabei ein hohes Maß an Sicherheit und Verlässlichkeit bietet.

Dabei kann aus Sicherheits- oder Dokumentationsgründen auch eine Authentifizierung des Arztes verlangt werden, beispielsweise durch einen Schlüssel, ein Paßwort, einen Barcode, eine Magnetkarte, einen, auch automatisch lesbaren, Chip, biometrische Daten, wie den Fingerabdruck oder den Augenabstand, oder eine sonstige, den Arzt individuell identifizierende Maßnahme. Durch diese Maßnahme wird gewährleistet, dass das Bestätigungselement nur von einer dem System bekannten und qualifizierten Person betätigt wird.

In einer besonders bevorzugten Ausführungsform ist das Bestätigungselement als Schaltfläche, beispielsweise mit Auswahlknöpfen, auf der Bedienoberfläche eines Touchscreens ausgebildet, der gleichzeitig als Ausgabevorrichtung der Parametereinstellungen zur Steuerung der Geräte dienen kann. Sofern vorgesehen ist, dass der Arzt die Parameter vor Übernahme durch die Geräte verändern kann, können die Werte der Parameter beispielsweise über Schiebeschalter oder Druckknöpfe auf der Bedienoberfläche des Touchscreens verändert werden. Diese Ausführungsform erlaubt eine besonders einfache, bequeme und übersichtliche Handhabung des gesamten Systems, wodurch auch dessen allgemeine Betriebssicherheit erhöht wird.

Zur Erhöhung der Sicherheit kann auch ein erstes Bestätigungselement vorgesehen sein, das zur Auswahl einzelner, vorgegebener oder geänderter Parameterwerte dient, und ein zweites Bestätigungselement, das erst nach Auswahl und Zustimmung zu den Parametereinstellungen mit dem ersten Bestätigungselement betätigt werden kann. Erst die Betätigung des zweiten Bestätigungselements ermöglicht dem Benutzer den Betrieb der Geräte mit den zuvor durch das erste Bestätigungselement ausgewählten Parametereinstellungen. Durch die Betätigung des zweiten Bestätigungselements werden die Parameterwerte erst freigegeben und das System sozusagen "scharf" gemacht. Diese Maßnahme erhöht die Sicherheit des Systems und beugt einem versehentlichen Betrieb der sicherheitsrelevanten Geräte mit ungeeigneten oder gar falschen Parametereinstellungen vor.

In einer weiteren bevorzugten Ausführungsform ist ein erstes Bestätigungselement vorgesehen zur Auswahl von Einstellungen und Parametern zur Steuerung nicht-sicherheitsrelevanter Geräte sowie ein zweites Bestätigungselement zur Auswahl von Einstellungen und Parametern zur Steuerung sicherheitsrelevanter Geräte.

Gemäß einer besonders bevorzugten Ausführungsform der Erfindung sind die vorbestimmten Gerätekonfigurationen und -parameter Teil einer Datenbank. Diese Datenbank kann insbesondere ein OP-Atlas oder eine OP-Wissensdatenbank sein, der eine Vielzahl von weiteren Daten enthalten kann, insbesondere lexikalische Daten, Bilddaten, eingriffsspezifische Anleitungen unter Angabe der einzelnen Operationsschritte und Warnhinweise für den ausführenden Arzt, Angabe der zu verwendenden Instrumente und Geräte sowie deren Einstellungen, usw.. Diese Informationen, die z. T. auch in der gedruckten Form eines OP-Atlas enthalten sind, sind in der Datenbank in digitalisierter Form abgelegt und können im Operationssaal vom Arzt aufgerufen und auf der Anzeigeeinheit dargestellt werden, um ihm für den jeweiligen Eingriff wesentliche Informationen zu übermitteln.

Durch diese Form der Datenbank hat der Arzt Zugriff auf Parametereinstellungen zur Steuerung der sicherheitsrelevanten Geräte, die für den jeweiligen medizinischen Eingriff angepasst und optimiert sind. Darüber hinaus kann er auf die sonstigen Informationen, die der digitalisierte OP-Atlas beinhaltet, zurückgreifen.

Die Datenbank kann in der Weise strukturiert sein, dass der Benutzer durch Eingabe von anwendungsspezifischen Schlagworten oder über eine Menüsteuerung, die von Fachgebieten und schrittweise immer genauer spezifizierte Anwendungen anzeigt, zu den für den jeweiligen Eingriff relevanten Daten gelangt. Beim Aufruf der zu einem bestimmten Eingriff gehörenden Bild- oder Textdaten können dann die dazugehörigen Gerätekonfigurationen und -parameter dargestellt und zur Bestätigung durch das Bestätigungselement angeboten werden.

Die Datenbank kann über einen Browser verfügen, mit dem zwischen verschiedenen Fachgebieten und Untergebieten in der Datenbank navigiert werden kann. Es kann auch eine Navigation bezüglich der operativen Zugangswege realisiert sein, die beispielsweise weiter nach Fachgebieten und Eingriffen untergliedert sein kann. Die Wahl des operativen Zugangswegs bestimmt beispielsweise die Erfordernisse an die Lichtverhältnisse im Operationssaal und die räumlichen Lage, Orientierung und Einstellung des Operationstischs. Die entsprechenden Konfigurationen und Parametereinstellung können entsprechend der Zugangswege zusammengefasst sein und angewählt werden. Durch Betätigen eines ersten Bestätigungselements kann dann ein bestimmter Zugangsweg ausgewählt werden. Dadurch kann eine bequeme Umschaltung zwischen verschiedenen operativen Zugangswegen, beispielsweise zwischen vaginalen oder laparoskopischen Zugangsweg, ermöglicht werden. In der weiteren Navigation der Datenbank kann die Auswahl der Gerätekonfigurationen und -parametereinstellungen zur Steuerung der Geräte, die zur Durchführung des spezifischen Eingriffs vonnöten sind, durch Betätigung eines zweiten Bestätigungselements getroffen werden.

Auch die in einer solchen Datenbank abgelegten optimierten Gerätekonfigurationen und -parametereinstellungen können nicht für alle in der Realität auftretenden Situationen, die beispielsweise während eines Eingriffs akut auftreten können oder mit den anatomischen Eigenheiten eines Patienten zusammenhängen können, optimal sein. Die dadurch erzwungenen Abweichungen vom vorgegebenen Ablauf können nicht alle vom Hersteller einer Datenbank antizipiert werden. Hierauf angemessen zu reagieren, insbesondere bei der Planung, Vorbereitung und Durchführung des medizinischen Eingriffs, ist Aufgabe des behandelnden Arztes.

Einige system- und situationsabhängige Einflüsse auf die notwendigen Konfigurationen und Parametereinstellungen können jedoch zumindest teilweise vorhergesehen werden. So können die Gerätekonfigurationen und optimalen Parameterwerte abhängig von Hersteller und Modell der verwendeten Geräte variieren, ebenso können die Daten des Patienten, wie beispielsweise Alter, Größe, Gewicht und vorhandene bzw. bereits erkannte Erkrankungen und anatomische Besonderheiten, veränderte Parameterwerte erfordern.

Soweit sich diese system- und situationsabhängige Einflüsse in vorhersehbarer Weise auf die optimalen Gerätekonfigurationen und
- parametereinstellungen auswirken, können diese in den vorgegebenen Daten, beispielsweise in der Datenbank, berücksichtigt werden. So kann etwa die Datenbank verschiedene Parametersätze für verschiedene Hersteller oder Modelle der zu verwendenden Geräte enthalten. In einer bevorzugten Ausführungsform der Erfindung wird dann bei Aufruf der eingriffsspezifischen Daten derjenige Datensatz angezeigt, der den tatsächlich verwendeten Geräten entspricht. Die hierfür benötigten Daten sind innerhalb der erfindungsgemäßen Vorrichtung gespeichert oder es kann beim Aufruf der eingriffsspezifischen Daten darauf zugriffen werden. Ebenso können verschiedene, patientendatenabhängige Datensätze gespeichert sein, wovon derjenige, der den in der OP-Steuerung oder in einem angeschlossenen Netzwerk verfügbaren Patientendaten entspricht, aufgerufen wird. Alternativ hierzu kann auch, anstatt beispielsweise für jede mögliche Kombination patientenspezifischer Daten jeweils einen eigenen Satz von Geräteparametern zu speichern, eine Formel zur Berechnung patientenabhängiger Parameter hinterlegt sein, aus der mit Hilfe der verfügbaren Patientendaten die angezeigten zu übernehmenden Parameterwerte ermittelt werden, beispielsweise auch durch Interpolation oder Extrapolation aus vorgegebenen Eckwerten.

In einer bevorzugten Ausführung der Erfindung ist die Datenbank mit den Parametereinstellungen zur Steuerung der Geräte in dem Speicher eines externen Servers installiert, auf den die erfindungsgemäße Vorrichtung beispielsweise über ein lokales Netzwerk oder das Internet zugreifen kann.

Durch diese Maßnahme kann die Datenbank mit den Parametereinstellungen zur Steuerung der Geräte besonders leicht überarbeitet und aktualisiert werden und gleichzeitig einer Vielzahl an Nutzern zur Verfügung gestellt werden, die beispielsweise von verschiedenen Operationssälen innerhalb eines Klinikums oder von verschiedenen Klinikstandorten auf der Welt auf die Parametereinstellungen zugreifen können. Dadurch wird die Optimierung der Durchführung medizinischer Eingriffe erleichtert und ein Beitrag zu deren Qualitätssicherung geleistet.

In einer weiteren bevorzugten Ausführung der Erfindung wird die Datenbank der Parametereinstellungen zur Steuerung der Geräte auf einer Steuerungseinheit des Systems installiert und in dem Speicher der Steuerungseinheit abgelegt. Durch diese Maßnahme wird der Bedarf an zusätzlichen Geräten, wie Lesegeräten, gering gehalten und dadurch die apparative Störanfälligkeit des Systems reduziert, wodurch die apparative Sicherheit erhöht wird.

In einer weiteren bevorzugten Ausführungsform der Erfindung ist der Speicher mit der Datenbank der Parametereinstellungen zur Steuerung der Geräte als rein lesbaren Datenträger, beispielsweise als read-only CD, CD-ROM oder DVD, ausgebildet. Eine Steuerungseinheit kann auf die Parametereinstellungen, die sich auf dem nur lesbaren Datenträger befinden, durch ein entsprechendes Lesegerät, wie beispielsweise ein CD-, CD-ROM- oder DVD-Laufwerk, zugreifen. Durch diese Ausführungsform wird eine flexible Handhabbarkeit gewährleistet. Der Zugriff des Systems auf verbesserte und überarbeitete Parametereinstellungen kann in Form aktualisierter Versionen der Datenbanken auf nur lesbaren Datenträger besonders leicht ermöglicht werden. Dabei kann die Datenbank Bestandteil der Software der Steuerungseinheit bzw. des Bus-Systems sein und dadurch bei jeder Aktualisierung der Betriebssoftware mit aktualisiert werden.

In einer weiteren bevorzugten Ausführungsform der Erfindung dient das Bestätigungselement nur zur Bestätigung der Parameterwerte einiger der bei dem betreffenden Eingriff verwendeten Geräte, insbesondere der der sicherheitsrelevanten Geräte. Hierdurch wird der Arzt von der Überprüfung von Daten, die für die Sicherheit des Patienten weniger relevant sind, entlastet.

In einer weiteren bevorzugten Ausführungsform des erfindungsgemä-ßen Systems ist die Steuerungseinheit derart eingerichtet, dass vor Freigabe der Parameterwerte Plausibilitätstests zumindest zur Steuerung sicherheitsrelevanter Einrichtungen vorgenommen werden. Wird eine Gruppe von Parametereinstellungen zur Durchführung einer spezifischen Prozedur aus der Datenbank angewählt, so werden diese Parametereinstellungen Plausibilitätstest unterworfen, die von Routinen, die auf einer Steuerungseinheit implementiert sind, durchgeführt werden. Beispielsweise können Drücke keine negativen Werte annehmen, so dass eine Überprüfung des Vorzeichens des numerischen Werts der Parametereinstellung Klarheit verschaffen kann. Die Werte von Parametereinstellungen aus Datenbanken können mit Fehlern behaftet sein, die beim Einpflegen großer Datenmenge durch fehlerhaften Übertrag der Werte entstehen können. Erweisen sich die angewählten Parametereinstellungen bei einem durchgeführten Plausibilitätstest als fehlerhaft, muss eine neue Anwahl an Parametereinstellungen erfolgen bzw. der fehlerbehafteten Wert durch Eingabe eines korrekten Wertes mittels einer Eingabevorrichtungen berichtigt werden. Die Durchführung von Plausibilitätstest der Parameterwerte stellt eine Maßnahme dar, die in vorteilhafter Weise die Sicherheit des Systems erhöht und die Handhabung des Systems erleichtert.

Ebenso kann die Steuerungseinheit derart eingerichtet sein, dass vor Freigabe der Parameterwerte Plausibilitätstests zur Prüfung auf Geräte- oder Bedienungsfehler durchgeführt werden. Wenn beispielsweise ein Gerät noch nicht an die Stromversorgung angeschlossen ist oder ein HF-Kabel fehlerhaft ist, kann dies automatisch erkannt und vor Freigabe der Parameterwerte angezeigt oder die Freigabe der Parameterwerte abgelehnt werden. Ebenso kann während des Betriebs der Geräte eine solche Prüfung stattfinden und das Ergebnis angezeigt werden, ggf. mit einem Warnsignal.

In einer weiteren Ausführungsform kann eine dynamische Anpassung der Gerätekonfigurationen und Parametereinstellungen zur Steuerung der sicherheitsrelevanten Geräte an die aktuellen Erfordernisse während des Eingriffs erfolgen. Vitalparameter, wie beispielsweise der Blutdruck, die Atemfrequenz, die Herzfrequenz, der Puls, der Sauerstoffgehalt des Blutes oder die Körpertemperatur, können vom Patienten während des medizinischen Eingriffs erfasst werden und mit hinterlegten Sollwerten bzw. -bereichen verglichen werden. Falls Abweichungen der gemessenen Werte von den Sollwerten bzw. -bereichen registriert werden, können durch die Steuereinheit des mindestens einen Geräts neue Gerätekonfigurationen und Parametereinstellungen berechnet werden, die auf der mindestens einen Anzeigeeinheit dargestellt werden. Der Arzt kann durch Betätigen des Bestätigungselements diesen der Situation angepassten Gerätekonfigurationen und Parametereinstellungen zustimmen.

Neben den Vitalparametern können auch Messwerte, die beispielsweise durch die zu steuernden Geräte erfasst werden, zur dynamischen Anpassung verwendet werden. Dabei kann auf die Informationen eines OP-Atlas bzw. einer OP-Wissensdatenbarilc zurückgegriffen werden. Diese Informationen können gemeinsam mit den erfassten Messwerten durch geeignete Algorithmen und Simulationen zur Bestimmung angepasster Gerätekonfigurationen und Parametereinstellungen verrechnet werden.

In jedem Fall aber muss der behandelnde Arzt oder eine am medizinischen Eingriff beteiligte, autorisierte Person seine bzw. ihre Zustimmung zu den dynamisch angepassten Gerätekonfigurationen und Parametereinstellungen durch Betätigen des Bestätigungselements geben.

In einer weiteren bevorzugten Ausführungsform ist eine zumindestens teilweise telemedizinische Fernsteuerung der erfindungsgemäßen Vorrichtung vorgesehen. Dabei ist das mindestens eine Bestätigungselement derart ausgestaltet, dass bei jeder Betätigung des Bestätigungselements eine Überprüfung der Identität der betätigenden Person erfolgt und die Übertragung der bestätigten Gerätekonfigurationen und Parametereinstellungen nur bei positiver Authentifizierung der betätigenden Person erfolgt.

Diese Maßnahme hat den Vorteil, dass spezialisierte Ärzte aus der Ferne nicht nur beratend dem medizinischen Eingriff beiwohnen können, sondern einen unmittelbareren Einfluss auf den medizinischen Eingriff ausüben können.

Aus ergonomischen Gründen ist es oft wünschenswert, die Steuerung aller während einer Operation benötigten Systeme von einer zentralen Stelle, möglichst aus dem sterilen Bereich, fernsteuern bzw. bedienen zu können. Eine solche Steuerung kann z.B. durch ein Touch-Screen (mit sterilem Überzug) oder mit einer Sprachsteuerung erfolgen. Zu den gesteuerten Einrichtungen bzw. Systemen können beispielsweise endoskopische Geräte gehören, sowie OP-Tisch, OP-Beleuchtung, Anästhesieeinheiten, Geräte zur Zustandsüberwachung von Patienten, Beatmungsgeräte, Raumbeleuchtung, Klimaanlage, Telefon, Pager, Internet, Krankenhaus-Informationssystem, Verbrauchsmaterial, Verwaltungssystem und andere. Diese können von einer einzigen Steuerungseinheit gesteuert werden, wobei die einzelnen Geräte beispielsweise über einen CAN-Bus verbunden sind und die Steuerungseinheit als Master und die Geräte als Slaves arbeiten.

Der Nachteil einer solchen Vernetzung besteht insbesondere darin, dass der Software- und Hardware-Aufwand für die einzige Steuerungseinheit sehr hoch ist, da er an das zu steuernde Gerät mit den höchsten Sicherheitsanforderungen angepasst sein muss. Bei zu steuernden Geräten, die nicht diese hohen Sicherheitsanforderungen erfüllen müssen, geht dadurch möglicherweise Flexibilität oder die Einfachheit der Bedienung verloren. Würde jedoch die einzige Steuerungseinheit im Hinblick auf Sicherheitsaspekte nach weniger strengen Maßstäben aufgebaut, so bestünde die Gefahr (z.B. bei einem PC mit Standard-Software), dass sicherheitsrelevante Funktionen durch unzuverlässige Funktionen der nicht-sicherheitsrelevanten Systeme gefährdet würden.

Die bei einem medizinischen Eingriff, insbesondere bei einer chirurgischen Operation, verwendeten Geräte können in zwei unterschiedliche Gruppen eingeteilt werden: Einerseits sicherheitsrelevante Geräte wie z.B. endoskopische Lichtquelle, Insufflator, Spül- und Saugpumpe, HF-Generator, OP-Tisch-Steuerung etc., also Geräte, bei denen ein Ausfall oder Fehler für den Patienten lebensbedrohlich sein könnte; andererseits nicht-sicherheitsrelevante oder auch nicht-medizinische Geräte, wie Jalousie, Bildarchivierung, Raumbeleuchtung, Telefon, Klimaanlage, Pager, Internet, Materialverwaltung usw..

Gemäß einer besonders bevorzugten Ausführungsform der Erfindung ist daher eine zweite Steuerungseinheit vorgesehen, die mit der ersten Steuerungseinheit zum Datenaustausch verbunden ist. Die erste Steuerungseinheit ist zur Steuerung zumindest derjenigen Einrichtungen, die sicherheitsrelevante Funktionen ausführen, als geschlossenes System ausgebildet, das nicht durch den Benutzer konfiguriert werden kann, während die zweite Steuerungseinheit als offenes System zur Steuerung der übrigen Geräte ausgebildet ist.

Hierdurch wird die Sicherheit gegenüber ungewollten Fehlfunktionen erhöht, ohne die Flexibilität des Gesamtsystems hierdurch zu beschränken. Dadurch, dass auf der zweiten Steuerungseinheit beispielsweise auch Software zum Einsatz kommen kann, die nicht die strengen Sicherheitskriterien zur Steuerung sicherheitsrelevanter Einrichtungen erfüllen muss, kann auf kostengünstigere Standard-Software zurückgegriffen werden.

Dadurch, dass die erste Steuerungseinheit, die für die Steuerung der sicherheitsrelevanten Einrichtungen zuständig ist, als geschlossenes System ausgebildet ist, ist sichergestellt, dass Manipulationen am Betriebssystem oder an den Applikationen zur Steuerung der sicherheitsrelevanten Einrichtungen unmöglich sind. Daneben können auf der ersten Steuerungseinheit auch Applikationen zur Steuerung nicht-sicherheitsrelevanter Einrichtungen ablaufen, sofern diese Applikationen zuvor unter Sicherheitsaspekten getestet wurden und sichergestellt ist, dass diese keinen Einfluss nehmen auf die Steuerung der sicherheitsrelevanten Einrichtungen.

Vorteilhafterweise sind die beiden Steuerungseinheiten jeweils Bestandteile eigenständiger Rechner (PC). Es ist jedoch auch möglich, die beiden Steuerungseinheiten in einen Rechner zu integrieren, der über zumindest zwei Prozessoren (CPU) verfügt, wobei jeweils eine Steuerungseinheit von einem Prozessor realisiert wird.

Dabei sind die erste Steuerungseinheit und die sicherheitsrelevanten Einrichtungen vorzugsweise über ein Bus-System miteinander verbunden. Vorzugsweise sind die nicht-sicherheitsrelevanten Einrichtungen und die zweite Steuerungseinheit über ein weiteres Bus-System miteinander verbunden, wobei die beiden Bus-Systeme vorzugsweise unterschiedlich sind. Für das weitere Bus-System kann daher auf gängige Standard-Bus-Systeme zurückgegriffen werden. Das aufwendigere Bus-System der ersten Steuerungseinheit wird also nur für die Verbindung mit den sicherheitsrelevanten Einrichtungen eingesetzt.

Gemäß einer bevorzugten Weiterbildung ist eine Schnittstelleneinheit vorgesehen, die einerseits mit den beiden Steuerungseinheiten und andererseits mit Peripheriegeräten verbunden ist und jeweils eine der beiden Steuerungseinheiten mit den Peripheriegeräten verbindet. Bevorzugt wird die Schnittstelleneinheit über eine Steuerleitung von der ersten Steuerungseinheit gesteuert. Weiter bevorzugt zählen zu den Peripheriegeräten eine Anzeigeeinrichtung und/oder eine Eingabeeinrichtung, vorzugsweise eine Tastatur und eine Maus. Weiter bevorzugt ist die Anzeigeeinrichtung als Touch-Screen ausgebildet, so dass auch Eingaben darüber möglich sind.

Diese vorgenannten Maßnahmen führen zu dem Vorteil, dass einerseits die Kosten für das Gesamtsystem reduziert werden und andererseits die Bedienbarkeit deutlich vereinfacht wird, da die zur Eingabe von Steuerungsbefehlen oder zur Überwachung von Parametern erforderlichen Peripheriegeräte nur einmal vorhanden sein müssen. Der Operateur muss folglich nicht mehrere Anzeigeeinrichtungen im Blick haben. Ferner gewährleistet die Steuerleitung zwischen Schnittstelleneinheit und erster Steuerungseinheit, dass die die wichtigen sicherheitsrelevanten Einrichtungen steuernde Steuerungseinheit auch im Falle eines Ausfalls bzw. eines Fehlers der zweiten Steuerungseinheit die entsprechenden erforderlichen Funktionen zulässt und die wichtigen sicherheitsrelevanten Parameter auf dem Touch-Screen darstellt. Insgesamt wird also eine Erhöhung der Sicherheit des Systems erreicht.

In einer bevorzugten Weiterbildung weist die zweite Steuerungseinheit ein Empfangsmittel auf, um Fehlermeldungen von der ersten Steuerungseinheit zu erfassen und auf einem der Peripheriegeräte darzustellen. Dies hat den Vorteil, dass auch im Falle einer Verbindung der zweiten Steuerungseinheit zur Steuerung nicht sicherheitsrelevanter Einrichtungen mit der Schnittstelleneinheit Fehlermeldungen bezüglich sicherheitsrelevanter Einrichtungen dem Benutzer des Systems sofort übermittelt werden. Damit lässt sich vermeiden, dass die Darstellung solcher Fehlermeldungen erst beim erneuten Umschalten der Verbindung von erster Steuerungseinheit zu Peripheriegeräten dem Benutzer gemeldet wird. Dies hat folglich den Vorteil, dass die Sicherheit des Gesamtsystems weiter erhöht wird.

Es ist weiter bevorzugt, die beiden Steuerungseinheiten über einen Ethernet-Bus (TCP/IP-Protokoll) miteinander zu verbinden, da sich dieses Bus-System als zuverlässiges, kostengünstiges System herausgestellt hat.

Bevorzugt weist die erste Steuerungseinheit ein eingebettetes Betriebssystem, vorzugsweise "embedded Windows NT", auf, das gegenüber Eingriffen von außen geschützt ist. Damit ist das Betriebssystem der ersten Steuerungseinheit fester Bestandteil der Einheit und damit gegenüber Manipulationen geschützt. Der Benutzer kann keine Eingriffe in das Betriebssystem vornehmen, wie dies beispielsweise bei gängigen PCs möglich wäre. Damit wird vermieden, dass durch beabsichtigte oder unbeabsichtigte Eingriffe in das Betriebssystem bestimmte sicherheitsrelevante Funktionen nicht mehr oder fehlerhaft ausgeführt werden.

In einer bevorzugten Weiterbildung weist die erste Steuerungseinheit ein Prüfmittel auf, das die Verbindung zu der Schnittstelleneinheit zyklisch prüft und eine Fehlermeldung abgibt, wenn eine Verbindung nicht vorhanden ist. Auch diese Maßnahme führt zu einer Steigerung der Sicherheit, da das System dem Benutzer sofort mitteilen kann, wenn eine Darstellung bzw. eine Einstellung bestimmter Parameter sicherheitsrelevanter Einrichtungen aufgrund einer fehlerhaften Schnittstelleneinheit nicht mehr möglich ist.

In einer bevorzugten Weiterbildung umfasst die erste Steuerungseinheit eine Sprachsteuerungseinheit, beispielsweise in Form eines Softwaremoduls. Diese Maßnahme hat den Vorteil, dass sich die Bedienung für den Operateur vereinfacht und problemlos aus dem sterilen Bereich heraus möglich ist.

In einer bevorzugten Weiterbildung kommunizieren die beiden Steuerungseinheiten miteinander, wobei insbesondere die erste Steuerungseinheiten die zweite Steuerungseinheit auf Fehler überprüft. Es ist weiter bevorzugt, eine Schnittstelleneinheit vorzusehen, die von der ersten Steuerungseinheit gesteuert wird und abhängig davon Signale entweder von der ersten oder der zweiten Steuerungseinheit an gemeinsame Peripheriegeräte weiterleitet. Diese Maßnahme hat - wie bereits erwähnt - den Vorteil, die Kosten des Systems zu reduzieren und die Übersichtlichkeit der Bedienung und den Bedienungskomfort insgesamt zu steigern.

In einer bevorzugten Weiterbildung wird die erste Steuerungseinheit bei einem Fehler der zweiten Steuerungseinheit die Schnittstelleneinheit so ansteuern, dass die Signale der ersten Steuerungseinheit an die Peripheriegeräte weitergeleitet werden. Dies heißt, dass die erste Steuerungseinheit gewährleistet, dass ein Fehler in der zweiten Steuerungseinheit nicht zu einem Ausfall der Verbindung von erster Steuerungseinheit zu Peripheriegeräten führen kann.

In einer bevorzugten Weiterbildung leitet die Schnittstelleneinheit die Signale der ersten Steuerungseinheit an die Peripheriegeräte sofort weiter, wenn eine sicherheitsrelevante Funktion ausgeführt werden soll. Dies hat den Vorteil, dass die wichtigen Funktionen auch bei zunächst nicht vorhandener Verbindung zwischen der zweiten Steuerungseinheit und den Peripheriegeräten möglich sind. Eine Erhöhung der Sicherheit ist die Folge.

In einer bevorzugten Weiterbildung leitet die Schnittstelleneinheit nach dem Aktivieren einer nicht-sicherheitsrelevanten Funktion die Signale der zweiten Steuerungseinheit an die Peripheriegeräte erst dann weiter, wenn die sicherheitsrelevante Funktion vollständig ausgeführt bzw. abgearbeitet ist. Das heißt mit anderen Worten, dass die Ausführung sicherheitsrelevanter Funktionen nicht durch Umschalten der Schnittstelleneinheit unterbrochen werden kann. Vielmehr wird die Ausführung der sicherheitsrelevanten Funktion bis zum Ende ausgeführt, und erst dann wird die Schnittstelleneinheit die Verbindung zwischen zweiter Steuerungseinheit und Peripheriegeräten herstellen.

Ein erfindungsgemäßes System zur Durchführung medizinischer Eingriffe umfaßt eine erfindungsgemäße Vorrichtung zur Steuerung und/oder Überwachung mindestens eines Geräts bei einem medizinischen Eingriff, sowie mindestens ein solches Gerät, das einerseits mit der Steuerungseinheit bzw. den Steuerungseinheiten verbunden ist, und das andererseits mit mindestens einem Eingriffselement zur Verwendung bei dem medizinischen Eingriff verbunden ist. Das Eingriffselement kann beispielsweise ein Insufflationsschlauch sein, der wiederum mit einem Trokar verbunden sein kann, durch den Insufflationsgas in den Operationsraum geleitet wird, oder auch beispielsweise ein HF-chirurgisches Instrument.

Die der Erfindung zugrundeliegende Aufgabe wird auch durch ein Verfahren mit den Merkmalen des Anspruchs 16 gelöst, wodurch die bereits im Zusammenhang mit der erfindungsgemäßen Vorrichtung beschriebenen Vorteile verwirklicht werden.

Weitere Aspekte der Erfindung ergeben sich aus der nachfolgenden Beschreibung eines bevorzugten Ausführungsbeispiels und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

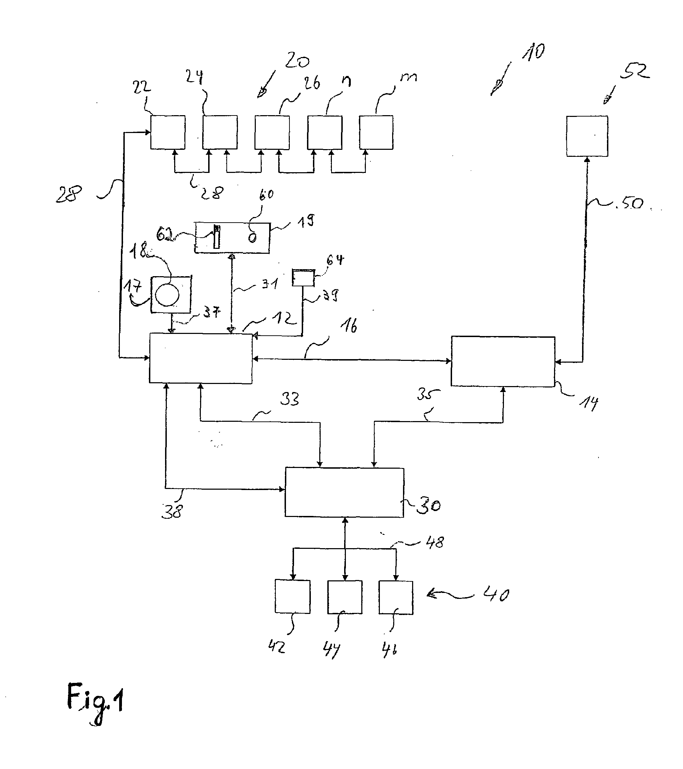

In Fig. 1 ist ein System zur zentralen Steuerung von Einrichtungen, die während einer Operation benutzt werden, in Form eines Blockschaltdiagramms dargestellt und mit dem Bezugszeichen 10 gekennzeichnet. Das System 10 umfasst eine erste Steuerungseinheit 12 und eine zweite Steuerungseinheit 14. Beide Steuerungseinheiten 12, 14 sind über eine Bus-Verbindung 16, beispielsweise eine Ethernet-Bus-Verbindung, miteinander verbunden, um Daten in Form von Nachrichten austauschen.

Beide Steuerungseinheiten 12, 14 sind als medizinische PCs ausgebildet, wobei in der ersten Steuerungseinheit 12 ein eingebettetes Betriebssystem, vorzugsweise ein "embedded Windows NT"- Betriebssystem, zum Einsatz kommt. Die zweite Steuerungseinheit 14 arbeitet vorzugsweise mit einem üblichen Windows- oder einem anderen nicht-eingebetteten Betriebssystem.

Die erste Steuerungseinheit 12 dient zumindest zur Steuerung von medizinischen Einrichtungen, die sicherheitsrelevante bzw. sicherheitskritische Funktionen ausführen. In der Figur sind diese sicherheitsrelevanten Geräte mit dem Bezugszeichen 20 gekennzeichnet. Beispielhaft sind in der Figur eine Pumpe 22, ein Insufflator 24 und ein HF-Generator 26 gezeigt. Diese beispielhafte Aufzählung dreier Geräte ist jedoch nicht einschränkend zu verstehen, was in der Figur durch weitere Geräte n und m angedeutet wird. Darüber hinaus kann die erste Steuerungseinheit 12 auch zur Steuerung nicht-sicherheitsrelevanter Geräte bei Verwendung entsprechend getesteter Software eingesetzt werden. Im folgenden wird jedoch auf diese Möglichkeit nicht weiter eingegangen.

Die Kommunikation zwischen der ersten Steuerungseinheit 12 und den sicherheitsrelevanten Geräten 20 erfolgt über ein Bus-System 28, das eine sichere Übertragung von Daten ermöglicht. Bei diesem Bus-System 28 sind andere Kriterien bezüglich Fehlersicherheit als beispielsweise bei dem vorgenannten Ethernet-Bus 16 anzulegen.

Das System 10 umfasst ferner eine Umschalteinheit 30. Die Umschalteinheit 30 ist eingangsseitig mit der Steuerungseinheit 12 und der Steuerungseinheit 14 verbunden, wobei in der Figur beispielhaft nur jeweils eine Verbindungsleitung 33, 35 dargestellt ist. Es versteht sich, dass es sich bei den beiden Verbindungen 33, 35 um eine Vielzahl von einzelnen Verbindungsleitungen handelt.

Ausgangsseitig ist die Umschalteinheit 30 mit Peripheriegeräten 40 verbunden, wobei stellvertretend in der Figur ein berührungsempfindlicher Monitor 42 (Touch-Screen), eine Eingabetastatur 44 sowie eine Maus 46 gezeigt sind. Die Peripheriegeräte 40 befinden sich beispielsweise im direkten Umfeld eines Operateurs oder eines Anästhesisten im Operationssaal, so dass diese Peripheriegeräte 40 entsprechend den Anforderungen in diesem Bereich angepasst sein müssen. Der Touch-Screen 42 ist beispielsweise mit einem sterilen Überzug versehen.

Die Verbindung der Peripheriegeräte 40 mit der Umschalteinheit 30 erfolgt über entsprechende Leitungen 48, wobei auch hier der Einfachheit wegen nur jeweils eine Leitung stellvertretend für eine Vielzahl von Verbindungsleitungen dargestellt ist.

Die Umschalteinheit 30 hat nun die Aufgabe, die einzelnen Peripheriegeräte 42 bis 46 mit einer der beiden Steuerungseinheiten 12, 14 zu verbinden, so dass die Eingabe bzw. die Darstellung von Daten möglich wird.

Die Steuerung der Umschalteinheit 30 übernimmt die erste Steuerungseinheit 12, wobei über eine Steuerleitung 38 entsprechende Steuersignale an die Umschalteinheit 30 übermittelt werden können.

Die Steuerungseinheit 12 ist mit einem Fingerabdruckerkennungsgerät 64 über eine Verbindungsleitung 39 verbunden.

Bevor Änderungen an der Konfiguration oder den Parameterwerten durch den behandelnden Arzt vorgenommen werden können, muss der Arzt sich durch Auflegen beispielsweise seiner rechten Zeigefingerspitze auf das Fingerabdruckerkennungsgerät 62 identifizieren. Das Fingerabdruckerkennungsgerät scannt den Fingerabdruck und vergleicht diesen mit hinterlegten Fingerabdrücken. Falls der gescannte Fingerabdruck mit einem hinterlegten Fingerabdruck übereinstimmt, können Änderungen der Gerätekonfigurationen und Parameterwerte zur Steuerung sicherheitsrelevanter Einrichtungen durch die Steuereinheit 12 ausgelöst werden.

Die Steuerungseinheit 12 ist mit einem Datenlesegerät 17, beispielsweise einem CD-ROM-Laufwerk, über eine Verbindungsleitung 37 verbunden. In dem Datenlesegerät 17 befindet sich ein Datenträger 18, beispielsweise eine Kompakt Disk (CD), auf dem sich ein OP-Atlas mit einer Datenbank von Parametereinstellungen zur Steuerung sicherheitsrelevanter Einrichtungen befindet.

Die auf dem Datenträger 18 befindlichen Informationen können durch die Steuerungseinheit 12 über die Verbindungsleitung 37 ausgelesen und auf der Anzeigevorrichtung 42 dargestellt werden. Der behandelnde Arzt kann eine Auswahl der Parametervoreinstellungen zur Steuerung der sicherheitsrelevanten Einrichtungen 22, 24 und 24 treffen und gegebenenfalls die Parametervoreinstellungen durch Eingaben über den Touch Screen 42 oder über die Eingabetastatur 44 oder über die Maus 46 den Bedürfnissen des medizinischen Eingriffs anpassen.

Nach Auswahl der Parametereinstellungen zur Steuerung sicherheitsrelevanter Einrichtungen, die an der Anzeigevorrichtung 42 angezeigt werden, muss der behandelnde Arzt seine Auswahl durch Betätigen des Schalters 62 des Bestätigungselements 19 bestätigen. Erst das Umlegen des Schalters 62 bewirkt, dass ein Signal über die Verbindungsleitung 31 auf die Steuerungseinheit 12 übertragen wird, das die Übertragung der Parametereinstellungen von der Steuerungseinheit 12 über das Bus-System 28 auf die sicherheitsrelevanten Geräte 22, 24 und 26 initiiert. Die übertragenen Parameterwerte werden von den Geräten 22, 24 und 26 übernommen und der Zustand der Geräte entsprechend eingestellt.

Die Kontrollleuchte 60 zeigt durch ihr Leuchten an, ob die an der Anzeigevorrichtung 42 dargestellten Parameterwerte auf die sicherheitsrelevanten Geräte übertragen wurden. Falls die Kontrollleuchte 60 nicht leuchtet, signalisiert sie dem Benutzer, dass noch keine oder noch keine nach einer von der Steuerungseinheit 12 durchgeführten Plausibilitätsprüfung gültige Auswahl von Parametereinstellungen getroffen wurde.

Die zweite Steuerungseinheit 14 ist über einen optischen Bus 50 galvanisch getrennt mit Geräten 52 verbunden, die nicht-sicherheitsrelevante Funktionen ausführen. Hierzu gehören beispielsweise eine Telefon-Fernbedienung, die Raumbeleuchtung etc. Die Steuerung dieser nicht-sicherheitsrelevanten Geräte wird somit von der zweiten Steuerungseinheit 14 geleistet.

Wie bereits erwähnt, ist die erste Steuerungseinheit 12 mit einem eingebetteten Betriebssystem ausgestattet. Dies soll gewährleisten, dass Eingriffe bzw. Manipulationen am System von außen nicht möglich sind. Die erste Steuerungseinheit 12 ist vielmehr als geschlossenes System ausgebildet, auf dem im wesentlichen nur Tasks ablaufen, die für die Steuerung der sicherheitsrelevanten Geräte 20 erforderlich sind. Bei entsprechender getesteter Eignung können daneben auch Tasks ablaufen, die der Steuerung der nicht-sicherheitsrelevanten Geräte 52 dienen.

Die zweite Steuerungseinheit 14 ist dagegen als üblicher medizinischer PC ausgebildet. Im Gegensatz zu der ersten Steuerungseinheit 12 können auf der zweiten Steuerungseinheit 14 keine Tasks ablaufen, die der Steuerung sicherheitsrelevanter Geräte dienen.

Beide Steuerungseinheiten 12, 14 liefern Daten über die entsprechenden Leitungen 33, 35 an die Umschalteinheit 30, wobei je nach Zustand der Umschalteinheit nur die Daten einer der beiden Steuerungseinheiten auf dem Touch-Screen 42 dargestellt werden. Auch die Eingabe von Daten erfolgt dann nur in diese Steuerungseinheit. Möchte der Operateur beispielsweise Funktionen der anderen Gruppe von Geräten auswählen, kann er dies über die Eingabe eines entsprechenden Befehls tun, der entweder direkt von der ersten Steuerungseinheit 12 oder indirekt über die Steuerungseinheit 14 und den Bus 16 von der ersten Steuerungseinheit 12 empfangen wird. Diese sendet daraufhin ein entsprechendes Steuersignal über die Steuerleitung 38 aus, was ein Umschalten in der Umschalteinheit 30 zur Folge hat. Auf dem Touch-Screen 42 werden anschließend die entsprechenden Daten, Auswahlmenüs etc. der ausgewählten Gruppe von Geräten dargestellt.

Im Falle einer Verbindung der zweiten Steuerungseinheit 14 mit den Peripheriegeräten 40 ist es jedoch erforderlich, dass jegliche Fehlermeldungen, die sicherheitsrelevante Geräte 20 betreffen, sofort dem Operateur unabhängig von dem Schaltzustand der Umschalteinheit 30 über den Touch-Screen 42 mitgeteilt werden. In der zweiten Steuerungseinheit 14 läuft hierfür eine Task, die ständig die über den Bus 16 von der ersten Steuerungseinheit 12 gelieferten Nachrichten auf Fehlermeldungen überprüft. Bei Feststellen einer Fehlermeldung sorgt die zweite Steuerungseinheit 14 dafür, dass auf dem Touch-Screen ein Fenster geöffnet wird, in dem diese Fehlermeldung dargestellt wird.

Eine weitere Aufgabe der ersten Steuerungseinheit 12 besteht darin, das Vorhandensein der Umschalteinheit 30 zu überprüfen. Sollte die Umschalteinheit 30 beispielsweise durch Ausfall für die erste Steuerungseinheit 12 nicht mehr zu erkennen sein, muss sie sofort eine Fehlermeldung abgeben. Diese Fehlermeldung soll dem Operateur signalisieren, dass eine richtige Darstellung und eine Eingabe von Daten über die Peripheriegeräte 40 nicht mehr gewährleistet ist.

Ferner ist es notwendig, dass die erste Steuerungseinheit 12 die zweite Steuerungseinheit 14 überprüft und im Fehlerfall die Umschalteinheit 30 sofort in denjenigen Schaltzustand steuert, bei dem die erste Steuerungseinheit 12 mit den Peripheriegeräten 40 verbunden ist.

Unter Sicherheitsaspekten ist es zudem erforderlich, dass bei der Eingabe eines Befehls zur Umschaltung der Peripheriegeräte 40 auf die zweite Steuerungseinheit 14 zunächst alle noch nicht beendeten Funktionen der sicherheitsrelevanten Geräte 20 ausgeführt werden mit einer entsprechenden Darstellung der Parameter. Dies soll gewährleisten, dass die Ausführung dieser sicherheitsrelevanten Funktionen nicht zu früh beendet wird. Im umgekehrten Fall werden die Peripheriegeräte 40 jedoch sofort mit der ersten Steuerungseinheit 12 verbunden, so dass sicherheitsrelevante Funktionen ohne Wartezeit sofort ausgeführt werden können.

### Bezugszeichenliste

10 - System zur zentralen Steuerung von Einrichtungen
12 - erster Steuerungseinheit
14 - zweite Steuerungseinheit
16 - Busverbindung
17 - Lesegerät
18 - Datenbank/Speicher OP-Atlas
19 - Bestätigungselement
20 - sicherheitsrelevante Geräte
22 - Pumpe
24 - Insufflator
26 - HF-Generator
28 - Bus-System
30 - Umschalteinheit
31 - Verbindungsleitung
33 - Verbindungsleitung
35 - Verbindungsleitung
37 - Verbindungsleitung
38 - Steuerleitung
39 - Verbindungsleitung
40 - Peripheriegeräte
42 - berührungsempfindlicher Monitor (Touch Screen)
44 - Eingabetastatur
46 - Maus
48 - Leitungen
50 - optischer Bus
52 - nicht-sicherheitsrelevante Geräte
60 - Kontrollleuchte
62 - Schalter
64 - Fingerabdruckerkennungsgerät

## Patentansprüche

1. Vorrichtung zur Steuerung und/oder Überwachung mindestens eines Gerätes bei einem medizinischen Eingriff, umfassend:
- mindestens eine Steuerungseinheit zur Steuerung und/oder Überwachung des mindestens einen Gerätes und
- mindestens eine Anzeigeeinheit zur Anzeige von Daten,
- wobei ein Zugriff möglich ist auf eingriffsspezifische Daten, die zumindest Daten über die bei einem vorgegebenen Eingriff bei dem mindestens einen Gerät einzustellenden Parameterwerte umfassen,
**dadurch gekennzeichnet, dass**
- die Parameterwerte zumindest teilweise benutzerunabhängig vorbestimmt sind,
- die Parameterwerte von einem Benutzer zur Anzeige mittels der Anzeigeeinheit aufgerufen werden können,
- und dass ein Bestätigungselement vorgesehen ist, durch dessen Betätigung die vorbestimmten Parameterwerte für das mindestens eine Gerät übernommen werden.

2. Vorrichtung nach Anspruch 1, **dadurch gekennzeichnet, dass** der Benutzer nach Anzeige der benutzerunabhängig vorbestimmten Parameterwerte geänderte Parameterwerte zur Verwendung bei einem oder mehreren Eingriffen eingeben kann, und dass nach Bestätigung des Bestätigungselements die geänderten Parameterwerte für das mindestens eine Gerät übernommen werden.

3. Vorrichtung nach Anspruch 1 oder 2, **dadurch gekennzeichnet, dass** die eingriffsspezifischen Daten auch vorbestimmte Daten über die bei dem vorgegebenen Eingriff zum Einsatz kommende Gerätekonfiguration umfassen.

4. Vorrichtung nach einem der Ansprüche 1 bis 3, **dadurch gekennzeichnet, dass** die vorbestimmten oder geänderten Parameterwerte nach Betätigung des Bestätigungselements an das Gerät bzw. die Geräte, die der vorbestimmten Gerätekonfiguration entsprechen, übertragen werden.

5. Vorrichtung nach einem der Ansprüche 1 bis 3, **dadurch gekennzeichnet, dass** die vorbestimmten oder geänderten Parameterwerte vor Betätigung des Bestätigungselements an das Gerät bzw. die Geräte, die der vorbestimmten Gerätekonfiguration entsprechen, übertragen werden, und dass nach Betätigung des Bestätigungselements ein Aktivierungssignal zur Übernahme der Parameterwerte übertragen wird.

6. Vorrichtung nach einem der Ansprüche 1 bis 5, **dadurch gekennzeichnet, dass** die Betätigung des Bestätigungselements auch Vorgänge zu Dokumentation und/oder verwaltungsmäßige Vorgänge auslöst.

7. Vorrichtung nach einem der Ansprüche 1 bis 6, **dadurch gekennzeichnet, dass** das Bestätigungselement als Schalter ausgebildet ist und ein Kontrollelement vorgesehen ist, das bei Betätigung des Bestätigungselements ein Signal abgibt.

8. Vorrichtung nach einem der Ansprüche 2 bis 7, **dadurch gekennzeichnet, dass** das Bestätigungselement als Schaltfläche auf der Bedienoberfläche eines Touchscreens ausgebildet ist und der Touchscreen gleichzeitig zur Anzeige der Parameterwerte und zur Eingabe von geänderten Parameterwerten dient.

9. Vorrichtung nach einem der Ansprüche 1 bis 8, **dadurch gekennzeichnet, dass** die eingriffsspezifischen Daten in einer Datenbank gespeichert sind.

10. Vorrichtung nach einem der Ansprüche 1 bis 9, **dadurch gekennzeichnet, dass** die eingriffsspezifischen Daten situations- und/oder patientenspezifisch sind oder situations- und/oder patientenspezifisch angepaßt werden können.

11. Vorrichtung nach einem der Ansprüche 1 bis 10, **dadurch gekennzeichnet, dass** die Vorrichtung Speichermittel umfasst, auf denen die eingriffsspezifischen Daten gespeichert werden können.

12. Vorrichtung nach einem der Ansprüche 1 bis 11, **dadurch gekennzeichnet, dass** das Bestätigungselement nur zur Bestätigung der Parameterwerte der sicherheitsrelevanten Geräte dient.

13. Vorrichtung nach einem der Ansprüche 1 bis 12, **dadurch gekennzeichnet, dass** Überwachungsmaßnahmen zur Überprüfung der Plausibilität der Parameterwerte und/oder auf Fehler der Steuerungseinheiten oder Geräte durchgeführt werden.

14. Vorrichtung nach einem der Ansprüche 1 bis 13, **dadurch gekennzeichnet, dass** eine erste Steuerungseinheit zur Steuerung sicherheitsrelevanter Geräte und eine zweite Steuerungseinheit zur Steuerung nicht sicherheitsrelevanter Geräte vorgesehen sind.

15. System zur Durchführung medizinischer Eingriffe, umfassend
- eine Vorrichtung zur Steuerung und/oder Überwachung mindestens eines Geräts gemäß einem der vorhergehenden Ansprüche,
- mindestens ein Gerät zur Verwendung bei einem medizinischen Eingriff,
- das mit der Steuerungseinheit verbunden ist, und
- das mit mindestens einem Eingriffselement zur Verwendung bei dem medizinischen Eingriff verbunden ist.

16. Verfahren zur Steuerung und/oder Überwachung mindestens eines Gerätes bei einem medizinischen Eingriff, umfassend folgende Schritte:
- Bereitstellen des Zugriffs auf eingriffsspezifische Daten, die benutzerunabhängig vorbestimmte Daten über die bei einem vorgegebenen Eingriff bei dem mindestens einen Gerät einzustellenden Parameterwerte umfassen,
- Zugreifen auf die eingriffsspezifischen Daten nach Aufforderung durch einen Benutzer,
- Anzeigen der eingriffsspezifischen Daten, und
- Übernahme der eingriffsspezifischen Daten für das mindestens eine Gerät nach Betätigung eines Bestätigungselements durch den Benutzer.

17. Verfahren nach Anspruch 16, **dadurch gekennzeichnet, dass**
- nach Anzeige der eingriffsspezifischen Daten der Benutzer die Parameterwerte verändern kann, und dass
- nach Betätigung des Bestätigungselements durch den Benutzer die veränderten Parameterwerte für das mindestens eine Gerät übernommen werden.

## Geänderte Patentansprüche

### Geänderte Patentansprüche gemäss Regel 137(2) EPÜ.

**1.** Vorrichtung zur Steuerung und/oder Überwachung mindestens eines Gerätes (22, 24, 26, 52) bei einem medizinischen Eingriff, umfassend:
- mindestens eine Steuerungseinheit (12, 14) zur Steuerung und/oder Überwachung des mindestens einen Gerätes und
- mindestens eine Anzeigeeinheit (42) zur Anzeige von Daten,
- wobei ein Zugriff möglich ist auf eingriffsspezifische Daten, die zumindest Daten über die bei einem vorgegebenen Eingriff bei dem mindestens einen Gerät einzustellenden Parameterwerte umfassen,
**dadurch gekennzeichnet, dass**
- die Parameterwerte zumindest teilweise benutzerunabhängig vorbestimmt sind,
- die Parameterwerte von einem Benutzer zur Anzeige mittels der Anzeigeeinheit aufgerufen werden können,
- und dass ein Bestätigungselement (19) vorgesehen ist, durch dessen Betätigung die vorbestimmten Parameterwerte für das mindestens eine Gerät übernommen werden.

**2.** Vorrichtung nach Anspruch 1, **dadurch gekennzeichnet, dass** der Benutzer nach Anzeige der benutzerunabhängig vorbestimmten Parameterwerte geänderte Parameterwerte zur Verwendung bei einem oder mehreren Eingriffen eingeben kann, und dass nach Bestätigung des Bestätigungselements (19) die geänderten Parameterwerte für das mindestens eine Gerät übernommen werden.

**3.** Vorrichtung nach Anspruch 1 oder 2, **dadurch gekennzeichnet, dass** die eingriffsspezifischen Daten auch vorbestimmte Daten über die bei dem vorgegebenen Eingriff zum Einsatz kommende Gerätekonfiguration umfassen.

**4.** Vorrichtung nach einem der Ansprüche 1 bis 3, **dadurch gekennzeichnet, dass** die vorbestimmten oder geänderten Parameterwerte nach Betätigung des Bestätigungselements (19) an das Gerät bzw. die Geräte, die der vorbestimmten Gerätekonfiguration entsprechen, übertragen werden.

**5.** Vorrichtung nach einem der Ansprüche 1 bis 3, **dadurch gekennzeichnet, dass** die vorbestimmten oder geänderten Parameterwerte vor Betätigung des Bestätigungselements (19) an das Gerät bzw. die Geräte, die der vorbestimmten Gerätekonfiguration entsprechen, übertragen werden, und dass nach Betätigung des Bestätigungselements (19) ein Aktivierungssignal zur Übernahme der Parameterwerte übertragen wird.

**6.** Vorrichtung nach einem der Ansprüche 1 bis 5, **dadurch gekennzeichnet, dass** die Betätigung des Bestätigungselements (19) auch Vorgänge zu Dokumentation und/oder verwaltungsmäßige Vorgänge auslöst.

**7.** Vorrichtung nach einem der Ansprüche 1 bis 6, **dadurch gekennzeichnet, dass** das Bestätigungselement als Schalter (62) ausgebildet ist und ein Kontrollelement vorgesehen ist, das bei Betätigung des Bestätigungselements ein Signal abgibt.

**8.** Vorrichtung nach einem der Ansprüche 1 bis 8, **dadurch gekennzeichnet, dass** die eingriffsspezifischen Daten in einer Datenbank gespeichert sind.

**9.** Vorrichtung nach einem der Ansprüche 1 bis 9, **dadurch gekennzeichnet, dass** die eingriffsspezifischen Daten situations- und/oder patientenspezifisch sind oder situations- und/oder patientenspezifisch angepaßt werden können.

**10.** Vorrichtung nach einem der Ansprüche 1 bis 10, **dadurch gekennzeichnet, dass** die Vorrichtung Speichermittel (18) umfasst, auf denen die eingriffsspezifischen Daten gespeichert werden können.

**11.** Vorrichtung nach einem der Ansprüche 1 bis 11, **dadurch gekennzeichnet, dass** die Vorrichtung mindestens ein sicherheitsrelevantes Gerät und mindestens ein nicht sicherheitsrelevantes Gerät umfasst dass das Bestätigungselement (19) nur zur Bestätigung der Parameterwerte des mindestens einen sicherheitsrelevanten Geräts dient.

**12.** Vorrichtung nach einem der Ansprüche 1 bis 13, **dadurch gekennzeichnet, dass** die Vorrichtung mindestens ein sicherheitsrelevantes Gerät (22, 24, 26) und mindestens ein nicht sicherheitsrelevantes Gerät (52) umfasst und dass eine erste Steuerungseinheit (12) zur Steuerung des mindestens einen sicherheitsrelevanten Geräts und eine zweite Steuerungseinheit (14) zur Steuerung des mindestens einen nicht sicherheitsrelevanten Geräts vorgesehen sind.

**13.** System zur Durchführung medizinischer Eingriffe, umfassend
- eine Vorrichtung zur Steuerung und/oder Überwachung mindestens eines Geräts gemäß einem der vorhergehenden Ansprüche,
- mindestens ein Gerät zur Verwendung bei einem medizinischen Eingriff,
- das mit der Steuerungseinheit (12, 14) verbunden ist, und
- das mit mindestens einem Eingriffselement zur Verwendung bei dem medizinischen Eingriff verbunden ist.

**14.** Verfahren zur Steuerung und/oder Überwachung mindestens eines Gerätes bei einem medizinischen Eingriff, umfassend folgende Schritte:
- Bereitstellen des Zugriffs auf eingriffsspezifische Daten, die benutzerunabhängig vorbestimmte Daten über die bei einem vorgegebenen Eingriff bei dem mindestens einen Gerät einzustellenden Parameterwerte umfassen,
- Zugreifen auf die eingriffsspezifischen Daten nach Aufforderung durch einen Benutzer,
- Anzeigen der eingriffsspezifischen Daten, und
- Übernahme der eingriffsspezifischen Daten für das mindestens eine Gerät nach Betätigung eines Bestätigungselements durch den Benutzer.

**15.** Verfahren nach Anspruch 16, **dadurch gekennzeichnet, dass**
- nach Anzeige der eingriffsspezifischen Daten der Benutzer die Parameterwerte verändern kann, und dass
- nach Betätigung des Bestätigungselements (19) durch den Benutzer die veränderten Parameterwerte für das mindestens eine Gerät übernommen werden.
